(19) Europäisches Patentamt
European Patent Office
Office européen des brevets

(11) Veröffentlichungsnummer: **0 210 470 A2**

(12) # EUROPÄISCHE PATENTANMELDUNG

(21) Anmeldenummer: **86109182.5**

(22) Anmeldetag: **04.07.86**

(51) Int. Cl.⁴: **C07D 231/06**, C07D 231/54

(30) Priorität: **16.07.85 DE 3525268**

(43) Veröffentlichungstag der Anmeldung:
**04.02.87 Patentblatt 87/06**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Stroefer, Eckhard, Dr.**
**Werderstrasse 20**
**D-6800 Mannheim 1(DE)**
Erfinder: **Rohr, Wolfgang, Dr.**
**In der Dreispitz 13**
**D-6706 Wachenheim(DE)**
Erfinder: **Rotermund, Gerhard W., Dr.**
**Gluckstrasse 5**
**D-6800 Mannheim 1(DE)**
Erfinder: **Fischer, Rolf, Dr.**
**Bergstrasse 98**
**D-6900 Heidelberg(DE)**
Erfinder: **Neudert, Reinhard, Dr.**
**Suedring 35**
**D-6719 Bissersheim(DE)**

(54) **Verfahren zur Herstellung von 1-Pyrazolinen.**

(57) Verfahren zur Herstellung von 1-Pyrazolinen durch Oxidation von 1,3-Diaminen mit Natriumhypochlorit und Peroxid in wäßriger Lösung.

EP 0 210 470 A2

Xerox Copy Centre

## Verfahren zur Herstellung von 1-Pyrazolinen

Die Erfindung betrifft ein verbessertes, für den technischen Maßstab geeignetes Verfahren zur Herstellung von 1-Pyrazolinen durch Oxidation von 1,3-Diaminopropanen.

Es besteht ein Bedarf zur kostengünstigen Herstellung von 1-Pyrazolinen als Vorprodukt zu 2-Pyrazolinen, die wertvolle Heterocyclen-Bausteine im Pflanzenschutz-und Pharmabereich darstellen. Die Rohstoffbasis für die Synthese von 1-Pyrazolinen ist technisch und wirtschaftlich günstiger als die für die bisher bekannten Direktsynthesen für 2-Pyrazoline.

1-Pyrazoline sind bisher nur schwer und über Reaktionen mit geringen Ausbeuten (weit unter 30 %) zugänglich (A. Weissberger (Herausgeber), The Chemistry of Heterocyclic Cpds., Interscience Publ., New York-London-Sydney 1967, Bd. 22, L.C. Behr, R. Fuser, C.H. Jarboe Pyrazoles, Pyrazolines, Pyrazolidines etc., 177-208; R.J. Crawford, A. Mishra, R.J. Dummel, JACS 88 (1966), 3959). Sie sind thermodynamisch instabiler als die entsprechenden 2-Pyrazoline (A. Weissberger, loc. cit.) und zersetzen sich leicht in Stickstoff und Cyclopropane (A. Weissberger, loc. cit.; R.J. Crawford, A. Mishra, JACS 88 (1966), 3963).

Die Direktsysnthese des thermodynamisch stabileren 2-Pyrazolins aus Acroleinen und Hydrazinen (deutsche Patentanmeldung P 34 15 385.3) ist für technische Zwecke zu teuer.

Die Synthese von Pyrazolidin durch Umsetzung von Diaminopropan mit Natriumhypochlorit bei Temperaturen unter 0°C ist in der Literatur beschrieben (A. Lüttringhaus, J. Jander, R. Schneider, Chem. Ber. 92 (1959), 1756). Das Pyrazolidin wurde nicht als solches isoliert, sondern nach Umsetzung mit Benzolsulfochlorid als Derivat in einer Ausbeute von 33 % erhalten.

Aus 1,3-Dibrompropan und Hydrazin hergestelltes Pyrazolidin wurde zu 1-Pyrazolin dehydriert (R.J. Crawford, A. Mishra, R.J. Dummel, JACS 88 - (1966), 3959). Das Verfahren ist für technische Zwecke nicht geeignet.

Bei den Synthesen von Pyrazolinen und Pyrazolidinen aus Diaminen laufen in starkem Maße Folge-und Nebenreaktionen ab, wenn unter oxidativen Bedingungen gearbeitet wird. Dimere sind beobachtet worden (E.L. Buhle, A.M. Moore, F.Y. Wiselogle, JACS 65 (1943), 29). Alle Versuche in den zitierten Literaturstellen sind nur im Labormaßstab mit Kleinstmengen durchgeführt worden.

Die Umsetzung von Aminen mit chlorhaltigen Verbindungen unter oxidativen Bedingungen kann zur Explosion des Reaktionsgemisches führen - (Bildung von extrem instabilem $NCl_3$; Lehrbücher der anorgan. Chemie, z.B. A.F. Hollemann, E. Wiberg, Lehrbuch der anorgan. Chemie, W. de Gruyter Verlag, Berlin 1971, 80. Aufl., S. 349). Dieser Tatbestand hat im abschreckenden Sinne weitere Untersuchungen, vor allem im größeren Maßstab, behindert.

Der Erfindung lag die Aufgabe zugrunde, ein sicher zu handhabendes, technisch brauchbares Verfahren zur Herstellung von 1-Pyrazolinen zu entwickeln.

Die Lösung der Aufgabe besteht in einem Verfahren zur Herstellung von 1-Pyrazolinen durch Oxidation von 1,3-Diaminopropanen der Formel II

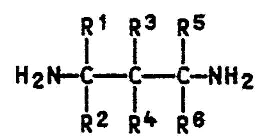

II,

wobei $R^1$ bis $R^6$ unabhängig voneinander Wasserstoff oder Alkyl mit 1 bis 3 Kohlenstoffatomen oder zwei der genannten Reste, die am gleichen oder an zwei einander benachbarten C-Atomen sitzen, gemeinsam einen Alkylenrest mit 3 bis 5 Kohlenstoffatomen bedeuten, in wäßriger Lösung, das dadurch gekennzeichnet ist, daß man das Diamin in 10-bis 30-, vorzugsweise 15-bis 25 %iger Lösung vorlegt und gleichzeitig eine wäßrige Lösung von Kalium-oder vorzugsweise Natriumhypochlorit mit einem Gehalt von 5 bis 22, vorzugsweise 15 bis 19 Gew.% $OCl^-$-Ionen und eine 20 bis 50, vorzugsweise 40 bis 50 gew.%ige wäßrige Lösung eines Oxidationsmittels der Formel III

$R^3$-O-O-H III,

wobei $R^3$ vorzugsweise Wasserstoff, aber auch einen Alkylrest mit 1 bis 5 C-Atomen oder einen aliphatischen Acylrest mit 2 bis 5 C-Atomen darstellt, im Molverhältnis Hypochlorit : III wie 0,5 bis 1,5, vorzugsweise 0,9 bis 1,1 unter Rühren langsam zulaufen läßt und dafür sorgt, daß die Reaktionstemperatur nicht über 50°C, vorzugsweise

nicht über 30°C steigt, möglichst bald nach vollständiger Umsetzung des Diamins das entstandene Pyrazolin mit einem Pyrazolin lösenden, hydrophoben organischen Lösungsmittel bei Raumtemperatur extrahiert und im Vakuum bei Temperaturen unter 100°C, vorzugsweise unter 50°C, destilliert. Man erhält dabei Ausbeuten an 1-Pyrazolin in der Größenordnung von 90 % und mehr. Das Verfahren ist kontinuierlich ohne Zwischenfälle (z.B. Explosion) durchgeführt worden. Statt Wasser als Reaktionsmedium könnte im Prinzip auch eine wäßrig-alkoholische Mischung verwendet werden, doch bringt das keine Vorteile.

Die Diaminlösung wird vorgelegt, damit sie nicht mit einem starken Überschuß an Oxidationsmittel in Berührung kommt, weil das zu Zersetzungen führen könnte. Aus dem gleichen Grund muß die Reaktionstemperatur kontrolliert werden. Die Hypochlorit-und die Peroxidlösung läßt man getrennt zulaufen, oder man kann sie auch unmittelbar vor der Zugabe miteinander mischen. Das Hypochlorit wird als kommerziell erhältliche wäßrige Lösung eingesetzt, ebenso die Peroxidlösung. Höhere Konzentrationen als 50 % sind bei Peroxidlösungen nicht kommerziell erhältlich, wesentlich geringere als 20 % würden zu einer unnötigen Verdünnung des Reaktionsansatzes führen. Als wasserlösliche Peroxide kommen u.a. in Betracht: tert.-Butylhydroperoxid, Peressigsäure und deren Homologe, sowie vorzugsweise Wasserstoffperoxid.

Die Zulaufgeschwindigkeit der Oxidationslösungen ist durch die Reaktionsgeschwindigkeit begrenzt, um eine Anreicherung an Oxidationsmittel im Reaktionsraum zu vermeiden. Man bleibt in der Reaktionsmischung sowohl mit der Hypochlorit-wie mit der Oxidationsmittelkonzentration unter 5, vorzugsweise unter 3 Gew.%.

Der Umsatz wird zweckmäßig gaschromatographisch und/oder kernresonanzspektroskopisch verfolgt. Es kommt darauf an, daß bei vollständigem Umsatz des Diaminopropans der Zulauf beider Oxidationsmittellösungen sofort abgebrochen und das Wertprodukt rasch aus dem Reaktionsgemisch abgetrennt wird. Die Umsetzungszeit unter gegebenen Bedingungen ermittelt man zweckmäßig in einem Vorversuch. Die Abtrennung geschieht durch Extraktion mit einem hydrophoben Lösungsmittel, das das 1-Pyrazolin gut löst und dessen Siedepunkt (zur Erleichterung der destillativen Trennung) weit von dem des 1-Pyrazolins (nach oben oder unten) entfernt ist. Beispiele hierfür sind: Aromatische und araliphatische Kohlenwasserstoffe, halogenierte Kohlenwasserstoffe, hydrophobe Heterocyclen, ferner N-Butylbenzolsulfamid. Bevorzugt werden Chinolin und Methylenchlorid. Das erste bleibt bei der destillativen Trennung im Sumpf zurück, das zweite destilliert über.

Aufgrund der lösungsvermittelnden Wirkung des 1-Pyrazolins enthält der Extrakt auch Wasser. Die Aufarbeitung erfolgt destillativ unter möglichst gutem Vakuum, mit anderen Worten bei möglichst tiefer Temperatur, zweckmäßig unter Einsatz eines Sambay-Verdampfers, um die thermische Belastung des Wertproduktes so gering wie möglich zu halten.

Man erhält so eine wäßrige Lösung des 1-Pyrazolins. Das Wasser könnte im Prinzip destillativ (über ca. 100 Böden) abgetrennt werden. Dies ist aber für praktische Zwecke weder erforderlich noch empfehlenswert, weil die Stabilität des 1-Pyrazolins mit steigender Konzentration sinkt. In der Regel bringt es keine Vorteile, eine höhere Konzentration an 1-Pyrazolin in Wasser als 40 bis 50 Gew.% einzustellen. Wäßrige Lösungen sind nur stabil, wenn sie frei von Salzen und von Peroxiden sind. Deshalb muß nach der Umsetzung rasch extrahiert werden.

Auf die beschriebene Weise sind nicht nur 1-Pyrazolin selbst, sondern beispielsweise auch folgende Derivate herstellbar: 4,4-Dimethyl-1-pyrazolin (aus Neopentandiamin); Bicyclo[3,3,0]1,2-diaza-3,3a,4,5,6,6a-hexahydro-pentalen IV (aus 2-Aminomethyl-cyclopentylamin)

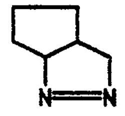

IV.

Die Aminogruppen müssen primär sein, brauchen aber nicht an einem primären Kohlenstoffatom zu sitzen. Allerdings soll das die Aminogruppe tragende Kohlenstoffatom keine sterisch aufwendigen (voluminösen) Reste tragen, die den Ringschluß behindern könnten.

Beispiel 1

In einem 6 l-Kolben mit Kühler, Rührer und zwei aufgesetzten Tropftrichtern wurden 200 g Diaminopropan in 1000 ml Wasser vorgelegt, 750 ml 50 %iges $H_2O_2$ in Wasser und 3000 ml einer wäßrigen NaOCl-Lösung (18 Gew.% Hypochloritionen) wurden gleichzeitig über 2,5 Stunden hinweg zugetropft. Die Temperatur im Kolben blieb unter

50°C. Das Reaktionsende wurde daran erkannt, daß alles Diaminopropan umgesetzt war (GC). Die Analyse des Reaktionsgemisches ergab eine Ausbeute von 95 % (quantitative GC und NMR). Das Gemisch wurde sofort in zwei Mixer-Settler-Stufen mit Chinolin bei Raumtemperatur kontinuierlich extrahiert (1 h Verweilzeit; Chinolin: Reaktionsgemisch = 1:1). Dabei wurde auch Wasser im Chinolin gelöst. Über einen kontinuierlich bei Raumtemperatur und 1,8 mbar Druck betriebenen Sambay wurden Wasser und 1-Pyrazolin aus dem Chinolin entfernt und in einer solegekühlten Vorlage aufgefangen. Das 1-Pyrazolin fiel als 20 %ige Lösung in Wasser an. In einer Kolonne hoher Trennstufenzahl wurden Wasser und 1-Pyrazolin getrennt. Die Gesamtausbeute an 1-Pyrazolin betrug 70 %. 1-Pyrazolin in wäßriger Lösung kann bevorzugt im schwach alkalischen Bereich in 90 %iger Ausbeute zu 2-Pyrazolin isomerisiert werden. Der diskontinuierliche Teil des Versuchs ist etwa 30 mal von verschiedenen Personen durchgeführt worden. Explosionen oder unstabile Zustände traten dabei nicht auf; das System war regelungstechnisch sicher zu fahren.

Beispiel 2

In einer Apparatur gemäß Beispiel 1, jedoch mit einem 15 l-Gefäß, wurden 300 g Diaminopropan in einem Gemisch von 800 ml Wasser und 200 ml Methanol vorgelegt, 2500 ml 30 %iges $H_2O_2$ in Wasser und 6000 ml einer wäßrigen NaOCl-Lösung (12 Gew.% Hypochlorit-Ionen) gleichzeitig über 3 h hinweg zugetropft. Die Temperatur im Kolben blieb unter 40°C. Analyse des Reaktionsgemisches (GC und NMR) ergab eine Ausbeute von 88 %. Das Gemisch wurde sofort in zwei Mixer Settler Stufen mit Methylenchlorid bei Raumtemperatur kontinuierlich extrahiert (80 min Verweilzeit; Methylenchlorid : Reaktionsgemisch = 1:2). Dabei wurde ebenfalls Wasser im Methylenchlorid gelöst. Über einen bei 41°C und Normal-Druck oder geringem Unterdruck betriebenen Sambay wurde das Methylenchlorid abgezogen. Das 1-Pyrazolin und Wasser verblieben im Sumpf (etwa 30 %ige Lösung). Die Gesamtausbeute an 1-Pyrazolin betrug 60 %.

Beispiel 3

Entsprechend Beispiel 1 wurde die gleiche molare Menge 2-Aminomethylcyclopentylamin eingesetzt. Der Reaktionsaustrag enthielt das Bicyclo-[3,3,0]1,2-diaza-3,3a,4,5,6,6a-hexahydropentalen in 80 %iger Ausbeute.

Beispiel 4

Entsprechend Beispiel 1 wurde Neopentandiamin eingesetzt. Der Reaktoraustrag enthielt das 4,4-Dimethyl-1-pyrazolin in 90 %iger Ausbeute. Nach Aufarbeitung gemäß Beispiel 1 ergab sich eine Gesamtausbeute von 65 %.

Vergleichsversuch 1 (mit 1,4-Diaminobutan statt 1,3-Diaminopropan)

Entsprechend Beispiel 1 wurde Diaminobutan eingesetzt. Das Gaschromatogramm des Reaktionsgemisches zeigt ein breites Spektrum verschiedener Verbindungen. Bei der Aufarbeitung konnte kein Diaza-Heterocyclus isoliert werden.

Vergleichsversuch 2 (nur mit NaOCl)

Entsprechend Beispiel 1 wurden 50 g (0,7 mol) Diaminopropan in 250 ml Wasser mit 1350 ml einer wäßrigen NaOCl-Lösung mit 18 Gew.% OCl- (4,7 mol) umgesetzt. Die Ausbeute an 1-Pyrazolin im Reaktionsaustrag fiel auf 40 %.

Vergleichsversuch 3 (nur mit $H_2O_2$)

Entsprechend Beispiel 1 wurden 200 g (2,8 mol) Diaminopropan in 1000 ml $H_2O$ mit 1200 ml 50 %iger (ca. 17 mol) wäßriger $H_2O_2$-Lösung umgesetzt. Die Ausbeute an 1-Pyrazolin im Reaktoraustrag fiel auf 35 %.

## Ansprüche

1. Verfahren zur Herstellung von 1-Pyrazolinen der Formel I

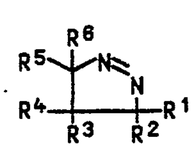

I

durch Oxidation von 1,3-Diaminopropanen der Formel II

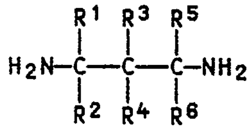

II,

wobei $R^1$ bis $R^6$ jeweils unabhängig voneinander Wasserstoff oder Alkyl mit 1 bis 3 Kohlenstoffatomen oder zwei der genannten Reste, die am gleichen oder an zwei einander benachbarten C-Atomen sitzen, gemeinsam einen Alkylenrest mit 3 bis 5 Kohlenstoffatomen bedeuten, in wäßriger Lösung, dadurch gekennzeichnet, daß man das Diamin in 10 bis 30 %iger Lösung vorlegt und gleichzeitig eine wäßrige Lösung von Hypochlorit mit einem Gehalt von 5 bis 22 Gew.% an Hypochlorit-Ionen und eine 20 bis 50 gew.%ige wäßrige Lösung eines Oxidationsmittels der Formel III

$R^3$-O-O-H III,

wobei $R^3$ Wasserstoff, einen Alkylrest mit 1 bis 5 Kohlenstoffatomen oder einen aliphatischen Acylrest mit 2 bis 5 Kohlenstoffatomen darstellt, im Molverhältnis Hypochlorit : III wie 1 : 0,5 bis 1,5 unter Rühren langsam zulaufen läßt und dafür sorgt, daß die Reaktionstemperatur nicht über 60°C steigt, möglichst bald nach vollständiger Umsetzung des Diamins das entstandene Pyrazolin mit einem Pyrazolin lösenden, hydrophoben organischen Lösungsmittel bei Raumtemperatur extrahiert und im Vakuum bei Temperaturen unter 100°C destilliert.

2. Verfahren nach Anspruch 1, dadurch gekennzeichnet, daß man eine 15 bis 19 Gew.% Hypochlorit-Ionen enthaltende wäßrige Natriumhypochloritlösung und eine 40 bis 50 %ige wäßrige Wasserstoffperoxidlösung so langsam zulaufen läßt, daß die Konzentration an Hypochlorit und Oxidationsmittel III im Reaktionsmedium jeweils 5 Gew.% nicht überschreitet.